Europäisches Patentamt

European Patent Office

Office européen des brevets

(19)

(11) Veröffentlichungsnummer: **0 175 989**
**B1**

(12) # EUROPÄISCHE PATENTSCHRIFT

(45) Veröffentlichungstag der Patentschrift:
14.06.89

(51) Int. Cl.⁴: **A 61 F 2/38**

(21) Anmeldenummer: **85111345.6**

(22) Anmeldetag: **07.09.85**

(54) Femurprothesenteil einer Kniegelenk-Endoprothese.

(30) Priorität: **26.09.84 DE 3435243**

(43) Veröffentlichungstag der Anmeldung:
**02.04.86 Patentblatt 86/14**

(45) Bekanntmachung des Hinweises auf die Patenterteilung:
**14.06.89 Patentblatt 89/24**

(84) Benannte Vertragsstaaten:
**AT BE CH FR GB IT LI LU NL SE**

(56) Entgegenhaltungen:
**DE-A- 2 728 427**
**DE-A- 2 744 710**

(73) Patentinhaber: **S + G IMPLANTS GMBH,
Grapengiesserstrasse 21, D-2400 Lübeck (DE)**

(72) Erfinder: **Grundei, Hans, Gärtnergasse 4, D-2400 Lübeck
(DE)**

(74) Vertreter: **Wilcken, Thomas, Dipl.-Ing. et al,
Musterbahn 1, D-2400 Lübeck (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf den Femurprothesenteil einer Kniegelenk-Endoprothese, der sich aus einem im Femurknochen zu verankernden Stiel und einem mit dem Stiel zu verbindenden Kufenteil zusammensetzt, wobei der Kufenteil aus zwei durch eine Brücke miteinander verbundenen Kufen zur Abstützung auf den Gleitflächen eines Tibiaprothesenteils besteht und die Brücke mit einem sich nach proximal verjüngenden Konuszapfen zum Eingriff mit Haftreibung in eine angepasste Bohrung des Stieles versehen ist.

Es ist bekannt, derartige Femurprothesenteile von Kniegelenk-Endoprothesen so auszubilden (DE-A 2 549 819), dass der in den Femurknochen einzuführende Stiel auf dem Aussenumfang z.B. mit ringförmigen Rippen versehen wird, die in Längsrichtung sägenzahnförmiges Profil besitzen. Es ist dadurch möglich, den Stiel als Einheit im Femurknochen besser zu verankern und mit ihm den sich auf Gleitflächen der Tibiaplateaus abstützenden Kufenteil durch einen oberen Konuszapfen, mit Haftreibung in eine entsprechende Bohrung des Stieles einsetzbar, zu verbinden, um dadurch einen Austausch des Tibiateiles zu ermöglichen.

Bisher war die Verankerung des Stieles im Femurknochen so, dass er sich nach längerer Zeit lösen konnte, und vor allem konnte der Stiel beim Einsetzen bzw. Einstossen in die Markhöhle des Femurknochens entweder zu tief oder nicht tief genug in der Markhöhle eindringen, so dass damit unter Umständen kein einwandfreier Sitz der Prothese erreicht werden konnte.

Die Aufgabe der Erfindung besteht darin, den Stiel des Femurprothesenteils einer Kniegelenk-Endoprothese in seiner Eindringtiefe in die Markhöhle des Femurknochens eindeutig festlegen zu können.

Die Aufgabe wird bei dem eingangs erwähnten Femurprothesenteil dadurch gelöst, dass der im Femurknochen vorteilhaft unlösbar zu verankernde Stiel die Verbindungsbrücke der Kufen seitlich gabelförmig übergreift und dass die Enden der Gabelarme Flansche aufweisen, die Anschläge gegen das Unterende des Femurknochens bilden und sich nach Eingriff des Konuszapfens in die Stielbohrung auf der proximalen Seite der Kufen abstützen.

Durch diese Lösung legen sich die Flansche an den Gabelenden des in die Markhöhle einzutreibenden Stieles gegen die vorbereitete ebene Abschlussfläche des Femurknochens, womit die Eindringtiefe des Stieles und damit die Lage des mit dem Stiel zu verbindenden Kufenteils eindeutig festgelegt ist.

Die Erfindung mit weiteren vorteilhaften Merkmalen wird nachstehend anhand der Zeichnung erläutert. Es zeigen:

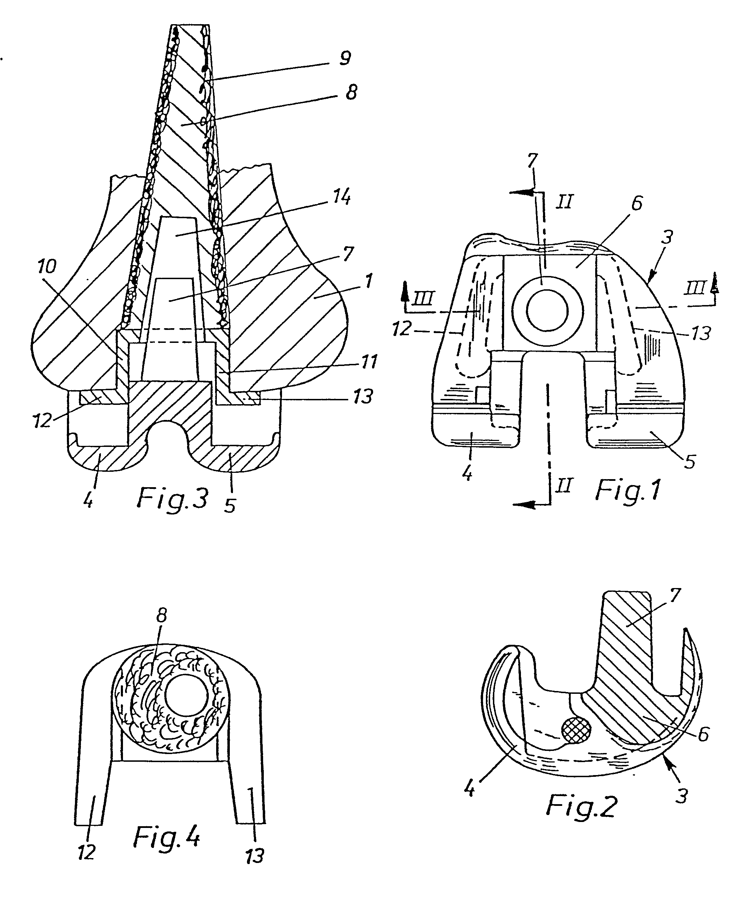

Figur 1 eine Aufsicht auf den die Kufen aufweisenden Teil des Femurprothesenteils einer Kniegelenk-Endoprothese,

Figur 2 einen Schnitt nach Linie II bis II der Fig. 1.

Figur 3 einen Schnitt nach Linie III bis III der Fig. 1, mit teilweisem Eingriff des Teiles nach Fig. 1 in einen im Femurknochen zu verankernden Stiel,

Figur 4 eine Aufsicht auf den im Femurknochen zu verankernden Stiel.

Der Femurprothesenteil einer Kniegelenk-Endoprothese setzt sich zusammen aus dem im Femurknochen 1 zu verankernden Stiel 8 und dem mit dem Stiel zu verbindenden Kufenteil 3. Der Kufenteil 3 besteht in bekannter Weise aus zwei Kufen 4 und 5, die sich auf Gleitflächen eines nicht dargestellten, bekannten Tibiaprothesenteils abstützen und die durch eine Brücke 6 miteinander verbunden sind. Die Brücke 6 ist mit einem sich nach oben konisch verjüngenden Befestigungszapfen 7 versehen.

Der in der Markhöhle des Knochens 1 zu verankernde metallische Stiel 8 ist vorteilhaft mit einer offenzelligen äusseren Metallschicht 9 versehen, deren Zellen das Einwachsen von Knochengewebe mit anschliessender Knochenbildung zulassen. Der Stiel kann aber auch eine glatte Oberfläche haben und wird dann einzementiert. Der Stiel 8 ist so ausgebildet, dass er am Unterende seitlich mit Gabelarmen 10, 11 über die Brücke 6 des Kufenteils 3 greift. Die Gabelarme 10, 11 sind an ihren Enden mit seitlichen Flanschen 12 und 13 versehen, die sich beim Eintreiben des Stieles 8 als Anschläge gegen die untere Schnittfläche des Femurknochens 1 legen und damit die Eindringtiefe des Stiels in die Markhöhle des Knochens begrenzen und festlegen. Anschliessend kann der Kufenteil 3 mit dem Stiel 8 verbunden werden, indem der Konuszapfen 7 mit Haftreibung in eine Bohrung 14 des Stieles 8 eingeführt wird, wobei sich die Flansche 12 und 13 auf den nach oben gerichteten Flächen der Kufen 4 und 5 abstützen. Damit ist die Verbindung des Femurprothesenteiles mit dem Femurknochen mit der Möglichkeit hergestellt, den Kufenteil 3 vom verankerten Stiel zu lösen, falls dies erforderlich wird.

## Patentanspruch

Femurprothesenteil einer Kniegelenk-Endoprothese, der sich aus einem im Femurknochen (1) zu verankernden Stiel (8) und einem mit dem Stiel zu verbindenden Kufenteil (3) zusammensetzt, wobei der Kufenteil (3) aus zwei durch eine Brücke (6) miteinander verbundenen Kufen (4, 5) zur Abstützung auf den Gleitflächen eines Tibiaprothesenteils besteht und die Brücke mit einem sich nach proximal verjüngenden Konuszapfen (7) zum Eingriff mit Haftreibung in eine angepasste Bohrung (14) des Stieles (8) versehen ist, dadurch gekennzeichnet, dass der im Femurknochen (1) vorteilhaft unlösbar zu verankernde Stiel (8) die Verbindungsbrücke (6) der Kufen (4, 5) seitlich gabelförmig übergreift und dass die Enden der Gabelarme (10, 11) Flansche (12, 13) aufweisen, die Anschläge gegen das Unterende des Femurknochens bilden und sich nach Eingriff des Konuszapfens (7) in die Stielbohrung (14) auf der proximalen Seite der Kufen (4, 5) abstützen.

## Claim

Femur prosthesis element of a knee joint endoprosthesis, which comprises a shaft (8) to be anchored in the femur bone (1) and a rocker portion (3) to be joined to the shaft, wherein the rocker portion (3) comprises two rockers (4, 5), joined together by a bridge (6), to be borne on the friction surfaces of a tibia prosthesis element, and the bridge is provided with a proximally tapering conical pin (7) for engagement under retaining friction in a matching bore (14) of the shaft (8), characterized in that the shaft (8) which is advantageously to be immovably anchored in the femur bone (1) laterally straddles the connecting bridge (6) of the rockers (4, 5) in a forked manner and that the ends of the fork arms (10, 11) comprise flanges (12, 13) which form stops against the lower end of the femur bone and bear on the proximal side of the rockers (4, 5) after engagement of the conical pin (7) in the shaft bore (14).

## Revendication

Partie fémorale d'une endoprothèse de l'articulation du genou, qui se compose d'une tige (8) devant être ancrée dans l'os (1) du fémur et d'un élément à patins (3) devant être relié à la tige, et dans lequel l'élément à patins (3) est formé de deux patins (4, 5) reliés entre eux par un étrier (6) et destiné à prendre appui sur les surfaces de glissement d'un élément de prothèse de tibia, et l'étrier comporte un embout conique (7), qui se rétrécit du côté proximal et est destiné à s'engager, de manière à y adhérer par frottement, dans un perçage adapté (14) de la tige (8), caractérisé en ce que la tige (8), qui avantageusement doit être ancrée de façon indétachable dans l'os (1) du fémur, enserre latéralement, à la manière d'une fourche, les patins (4, 5), et que les extrémités des branches (10, 11) de la fourche comportent des brides (12, 13), qui forment des butées s'appliquant contre l'extrémité inférieure de l'os du fémur et prennent appui sur le côté proximal des patins (4, 5), lorsque l'embout conique (7) est inséré dans le perçage (14) de la tige.

*Fig.3*

*Fig.1*

*Fig.4*

*Fig.2*